(19) Europäisches Patentamt
European Patent Office
Office européen des brevets

(11) EP 1 784 163 B1

(12) **EUROPEAN PATENT SPECIFICATION**

(45) Date of publication and mention of the grant of the patent:
**22.02.2012 Bulletin 2012/08**

(21) Application number: **05781134.1**

(22) Date of filing: **03.09.2005**

(51) Int Cl.:
***A61K 8/06*** *(2006.01)* ***A61K 8/67*** *(2006.01)*
***A61K 8/90*** *(2006.01)* ***A61K 9/107*** *(2006.01)*
***A61Q 19/00*** *(2006.01)* ***A61Q 19/08*** *(2006.01)*
***A61Q 19/02*** *(2006.01)* ***A23L 1/00*** *(2006.01)*
***B82Y 5/00*** *(2011.01)*

(86) International application number:
**PCT/KR2005/002929**

(87) International publication number:
**WO 2006/028339 (16.03.2006 Gazette 2006/11)**

(54) **NANO-EMULSION, THE USE THEREOF, AND PREPARATION METHOD THEREOF**

NANO-EMULSION, IHRE VERWENDUNG UND VERFAHREN ZU IHRER HERSTELLUNG

NANOEMULSION, SON UTILISATION ET SON PROCEDE DE PREPARATION

(84) Designated Contracting States:
**DE FR IT**

(30) Priority: **04.09.2004 KR 20040070665**

(43) Date of publication of application:
**16.05.2007 Bulletin 2007/20**

(73) Proprietors:
- **Kim, Young Dae Taechi-dong Kangnam-gu Seoul 135-280 (KR)**
- **Park, Kuen Ja Taechi-dong Kangnam-gu seoul 135-280 (KR)**
- **Kim, Jung Soo Taechi-dong Kangnam-gu Seoul 135-280 (KR)**
- **Kim, Ji Soo Taechi-dong Kangnam-gu Seoul 135-280 (KR)**

(72) Inventors:
- **Kim, Young Dae Taechi-dong Kangnam-gu Seoul 135-280 (KR)**
- **Park, Kuen Ja Taechi-dong Kangnam-gu seoul 135-280 (KR)**
- **Kim, Jung Soo Taechi-dong Kangnam-gu Seoul 135-280 (KR)**
- **Kim, Ji Soo Taechi-dong Kangnam-gu Seoul 135-280 (KR)**

(74) Representative: **Hubert, Philippe et al Cabinet Beau de Loménie 158, rue de l'Université 75340 Paris Cédex 07 (FR)**

(56) References cited:
**WO-A1-93/18752** **WO-A1-98/30205**
**WO-A1-99/26607** **WO-A2-02/26324**
**KR-A- 20010 017 595** **KR-A- 20020 091 858**
**KR-A- 20030 090 240** **US-A- 5 770 223**

- **MORISAKI K. ET AL: 'Design of novel hybrid vitamin C derivatives: thermal stability and biological activity' CHEM. PHARM. BULL. 1996, pages 1647 - 1655, XP001026205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 784 163 B1

## Description

[Technical Field]

The present invention relates to nanoemulsions, the use thereof, and preparation method thereof. More precisely, the invention relates to cosmetic compositions and their preparation methods of oil-in-water (o/w) nanoemulsions, which are prepared with polyoxypropylene-polyoxyethylene (POP-POE) vitamin E as emulsifiers and polyacrylic acid crosspolymers or polyacrylic acid derivative crosspolymers as emulsion assistants under the conditions of forming a complex of an oil phase and a water phase along with emulsifiers and emulsion assistants having a viscoelastic property at constant temperature (θ-point) by the proper control of the ratio of oil phase to oil phase plus water phase.

[Background Art]

Emulsion is a non-homogeneous system which one liquid is dispersed closely in another as a droplet. The mean droplet diameter of emulsion generally exceeds 0.1 μm but droplet diameters of emulsions in practical use are in the range of 0.5 to 100 μm. Emulsions have some demerits such as thermodynamic instability and poor absorption ability into the skin due to their large droplet size.

The smaller droplet size of emulsions not only suppresses the coalescence or coagulation of emulsion droplets but also suppresses the precipitation of emulsions and also helps to deliver the active agents into the skin.

But in the case of nanoemulsions, thermodynamic stability is improved very much and also the skin absorption ability of active agents is improved since the droplet size of nanoemulsions is much smaller than that of emulsions and the surface area of nanoemulsions is much larger than that of emulsions. The droplet size of nanoemulsions is defined differently depending on the author. In general the droplet size of nanoemulsions is defined as between 20 and 500 nm [Flockhart, I. R. etc, Nanoemulsions derived from lanolin show promising drug delivery properties, J. Pharm. Pharmacol. 50 (Supplement) 1998, 141].

There are generally two processes for production of nanoemulsions. The first and most common procedure is to use a high pressure homogenizer at high pressure along with surfactants and co-surfactants. The second procedure is to use the phase inversion temperature (PIT) principle.

But in the case of the procedure using a high pressure homogenizer there are some problems such as poor productivity and component deterioration due to difficult mass production and generation of much heat, respectively, since nanoemulsions should be prepared by contacting an oil phase with a water phase instantly through a narrow valve slit under a high pressure, for example, under 500-1.600 atmospheric pressure.

And since low viscosity emulsions can only be manufactured by this procedure high viscosity nanoemulsion creams can not be prepared because of the poor miscibility and aggregation of the phases. Therefore, by this method generally only oil in water liquid nanoemulsions of less than 20% oil phase can be prepared and cream nanoemulsions of high viscosity or hardness with a mean droplet diameter lower than 200 nm can not be prepared.

And in the case of the latter method of using a phase-inversion principle it is also difficult to control the PIT and the rapid cooling process during preparation. Also nanoemulsions prepared using the PIT principle have an instability problem of phase inversion near PIT since emulsions prepared by this method inverse at PIT easily.

Even though there are many problems described above nanoemulsions of low viscosity with a high amount of phospholipids as emulsifiers are mainly manufactured by using a high pressure homogenizer. And since the fluidity of droplets for instant contacting, mixing and the adsorption of surfactants at interfaces are important in the procedure of using a high pressure homogenizer, the use of polymeric thickeners like Carbomers which decrease the fluidity of droplets and may hinder the adsorption of surfactants at interfaces was avoided.

The production of very small droplets in emulsions requires a large amount of energy and needs to decrease the surface tension. Particularly, since there were not any good methods utilizing energy efficiently the emulsification method of using the inefficient high pressure homogenizer was mainly employed. But in this method a safety problem was triggered by the use of a large amount of surfactants. These problems were the main causes in limiting the practical application of nanoemulsions in cosmetics, drugs, etc.

In this context, after recognizing the importance of nanoemulsions in the industries of cosmetics, drugs, etc. the inventors studied nanoemulsions continuously and proposed the preparation method of nanoemulsion using POP-POE vitamin as emulsifiers (Korea patent no 488220), which can prepare nanoemulsions not only of low viscosity but also of high viscosity or hardness by simple stirring using a stirrer or a homogenizer at 1 atmospheric pressure. The preparation method described above is different from the difficult and inefficient conventional nanoemulsification methods of using a high-pressure homogenizer or a PIT principle. In the nanoemulsification method described above stable nanoemulsions can be prepared simply by the economical way of the "2 phase complex emulsification method" which consists of two steps of formation of high viscosity complex of an oil soluble phase and a water soluble phase of nanoemulsions by previous mixing and heating and then emulsification of the high viscosity complex by a high speed

propeller mixer. And the inventors acquired the certificate of Excellent New Technology(NT # 2002-027) entitled "Manufacturing Method of Antioxidative-Amphiphilic Tocopherol-based Nano Products" for the nanoemulsion technology described above from the Agency of Standards and Materials of Ministry of Commerce, Industry and Energy of the Korean Government. Afterwards, the inventors presented the study results of the nanoemulsion technology described above at the 22nd IFSCC Congress (Young-Dae Kim, etc. Novel Nanoemulsions of POP-POE Tocopheryl Ether, Edinburgh, October 14-17, 2002) and at the 23rd IFSCC Congress (Young-Dae Kim etc. A Novel Nanoemulsification Method: Stirring of a Mixture of Tocopherol-Containing Block Co-Polymer Emulsifier at θ-Point, Orlando, October 24-7, 2004) and the 3rd World Congress on Emulsion (Young-Dae Kim etc. The Formation and Stability of Liquid Nanoemulsions from Tocopherol Derivative Nonionic Amphiphiles, Lyon, September 24-27, 2002) and also published a paper in IFSCC Magazine (Young-Dae Kim, etc. A Novel Nanoemulsification Method of Stirring at the θ-Point with the Tocopherol-Based Block Copolymer Nonionic Emulsifier PPG-20 Tocophereth-50, vol 7, no. 4, Sept/Decem./2004 pp319-326).

The nanoemulsification method (Korea patent no 488220) described above was more economical than those using a high-pressure homogenizer or using a PIT principle and prepared excellent nanoemulsions of mean droplet diameter range of 206 to 455 nm.

The topical and transdermal nanoemulsions of WO93/18752 comprise a bioactive drug or cosmetic substance, a phospholipid emulsifier, a nonionic surfactant, toropherol succinate as anti-oxidant and crosslinked polyacrylate polymers as gelling agent and which has a mean droplet size from 0,05-0,5 microns.

[Disclosure]

[Technical Problem]

But the minimum droplet diameter of nanoemulsions prepared by the nanoemulsification method described above was 206 nm, which is a much larger value than 20 nm, the minimum droplet diameter of nanoemulsion defined. Therefore, improvement in the reduction of the droplet diameters is required.

One of the objects in this invention is to provide the O/W nanoemulsions of smaller droplet diameter with safety and stability by using a small amount of emulsifiers and emulsion assistants. Another object in this invention is to provide the cosmetic compositions, food compositions and drug compositions containing O/W nanoemulsions. And another object in this invention is to provide economical preparation methods of nanoemulsion compositions described above.

[Technical Solution]

In order to achieve the technical object in this invention, the inventors provide nanoemulsions containing polyoxypropylene-polyoxyethylene vitamin E as emulsifiers described in general formula 1: and polyacrylic acid crosspolymers or polyacrylic acid derivative crosspolymers as emulsion assistants (i.e. polyacrylic acids or their derivative crosspolymers).

[General formula 1]

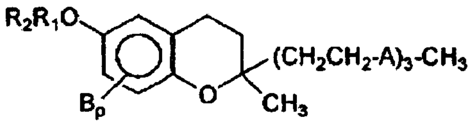

(1)

wherein, $R_1$ is $-(O-CH_2CH_2)_m-$ , m is an integer of 0 to 300, $R_2$ is $H(OCH(CH_3)CH_2)_n-$, n is an integer of 1 to 250; A is

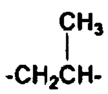

or $-C(CH_3)=CH-$; B is $-CH_3$ at the 5-, 7- or 8- position of vitamin E; and p is an integer of 1 to 3.

Of the total content of the nanoemulsions the content of polyoxypropylene-polyoxyethylene vitamin E is 0.5 to 60% by weight, preferably 1 to 30% by weight and that of polyacrylic acid or their derivative crosspolymers is 0.01 to 40% by weight, preferably 0.02 to 20% by weight and the remnant components are water soluble and oil soluble components for nanoemulsion compositions in this invention.

Another technical object in this invention is to produce nanoemulsion compositions containing the previously mentioned nanoemulsions.

Another technical object in this invention is to produce nanoemulsion topical treating compositions containing the previously mentioned nanoemulsions.

Another technical object in this invention is to create a preparation method of nanoemulsions, wherein oil soluble and water soluble components are mixed with the emulsifiers, polyoxypropylene-polyoxyethylene vitamin E described in general formula 1 and emulsion assistants, polyacrylic acid or their derivative crosspolymers.

Strictly speaking, the technical object is to provide the preparation method of nanoemulsion cosmetic compositions, wherein nanoemulsion cosmetic compositions of cosmetics, topical treatments and foods are obtained by the preparation of the viscoelastic complex of oil soluble phase and water soluble phase along with emulsifiers and emulsion assistants wherein, the weight ratio of oil soluble phase to oil soluble phase plus water soluble phase is in the range of 0.4 to 0.75 by using emulsifiers, polyoxypropylene-polyoxyethylene vitamin E described in general formula 1 and emulsion assistants, polyacrylic acid or their derivative crosspolymers at θ-Point of above 40°C, preferably above 50°C. The final nanoemulsification is then carried out after adding the remnant oil soluble or water soluble components if needed.

[General formula 1]

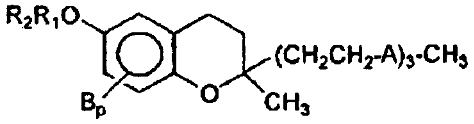

(1)

wherein, $R_1$ is $-(O-CH_2CH_2)_m-$, m is an integer of 0 to 300, $R_2$ is $H(OCH(CH_3)CH_2)_n-$, is an integer of 1 to 250; A is

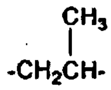

or $-C(CH_3)=CH-$; B is $-CH_3$ at the 5-, 7- or 8- position of vitamin E; and p is an integer of 1 to 3.

Nanoemulsions can be prepared by high-speed mixing since the complex of oil soluble and water soluble phase in this invention has a viscoelastic property above 40°C.
The above nanoemulsifications are carried out by using a disperser or a propeller or a homogenizer, etc.

[Advantageous Effects]

In this invention nanoemulsions containing POP-POE vitamin E as emulsifiers and polyacrylic acid or polyacrylic acid derivative crosspolymers as emulsion assistants have a very small mean droplet diameter ranging from 43 to 96 nm and are very stable and safe.

By the nanoemulsions described above the inventors obtained nanoemulsion cosmetic compositions with a much smaller mean droplet diameter than nanoemulsions prepared without emulsion assistants, polyacrylic acid or polyacrylic acid derivative crosspolymers by emulsifying using a simple high speed mixer such as a propeller, a disperser and a homogenizer due to the much improved properties when forming viscoelastic complex of oil soluble phase and water soluble phase above 40°C.

[Description of Drawings]

Figure 1 shows a microphotograph of a Cryogenic-Transmission Electron Microscope (Cryo-TEM) of nanoemulsion cosmetic composition in Example 6 in this invention, 1 day after preparation.
Figure 2 shows a Cryo-TEM microphotograph of nanoemulsion cosmetic composition in Comparative Example 6 in this invention. I day after preparation.
Figure 3 shows a microphotograph of an Atomic Force Microscope (AFM) of nanoemulsion cosmetic composition in Example 7 in this invention, I day after preparation.

Figure 4 shows an AFM microphotograph of nanoemulsion cosmetic composition in Comparative Example 7 in this invention, I day after preparation.

[Detailed Description of the Invention]

Hereinafter, the invention will be described in more detail.

O/W stable nanoemulsions of mean droplet diameter range of 43 to 96 nm having good productivity and safety due to the fact that they contain emulsifiers, polyoxypropylene-polyoxyethylene vitamin E described in general formula 1 and emulsion assistants, polyacrylic acid or their derivative crosspolymers, are provided in this invention.

[General formula 1]

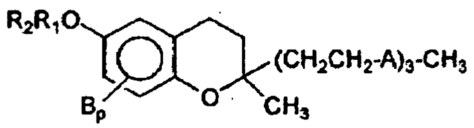

(1)

wherein, $R_1$ is $-(O-CH_2CH_2)_m-$, m is an integer of 0 to 300, $R_2$ is $H(OCH(CH_3)CH_2)_n-$, n is an integer of 1 to 250; A is

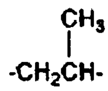

or $-C(CII_3)=CII-$; B is $-CH_3$ at the 5-, 7- or 8- position of vitamin E; and p is an integer of 1 to 3.

The above polyacrylic acid or polyacrylic acid derivative crosspolymers are homopolymers, Carbomers, obtained by the cross linking reaction of polyacrylic acid described in general formula 2 below with allylether of polyol or allylether of propylene or copolymers, acrylates/$C_6$-$C_{40}$ alkylacrylate crosspolymers prepared by the crosslinking reaction of $C_6$-$C_{40}$ alkylacrylates or more than 1 acrylic acid or methacrylic acid or their esters with allylether of polyol. In this invention, unlimited examples of allylethers of polyol could be used, but allylether of pentacrythritol or allylether of sucrose are suggested.

[General formula 2]

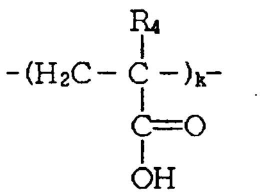

Wherein, $R_4$ is II or $CH_3$, k is an integer of 10 to 100,000.

The nanoemulsions described above are not limited to a particular use but are applicable to several industrial areas such as cosmetics, foods, topical treatments, etc.

Hereinafter, nanoemulsion cosmetic compositions containing nanoemulsions and their preparation method will be described precisely.

The emulsifiers in nanoemulsion compositions in this invention are POP-POE vitamin E as described in general formula 1 wherein, preferably m is an integer of 0 to 300 and m is an integer of 1 to 250, more preferably m is an integer of 20 to 150 and n is an integer of 10 to 100. POP-POE vitamin E in general formula 1 can be prepared by the preparation method in Korea Patent Laid-open publication No. 2000-0000840 or are commercially available.

Emulsion assistants, polyacrylic acids or their derivative crosspolymers are manufactured using the method described above or are commercially available. Of Carbomers, product names that are commercially available in the markets are, for example, Carbopol 910, -934, -940, -934p, -954, -961, -980, -9890, -981, -2984, -5984, -ETD2001, ETD2050, Carbopol Ultretz 10 of Goodrich company, Acritamcr 501E, -504E, -934, -943, -941 of Rita. Of acrylates/C6-C40 alkylacrylate crosspolymers, commercial products are, for example, Carbopol 1342, -1382, ETD 2020, Pemuten-TR-1, -TR-2 of Goodrich company.

On the other hand the principles of forming nanoemulsions investigated by the inventors in this invention are as follows.

The ratio of the depth of adsorption layer (8) of emulsifier to the diameter of droplet (R) in the nanoemulsion with a smaller droplet diameter is larger than that with a larger droplet diameter. Therefore, the repulsive force is very large and prevents the aggregation of the systems. For droplets to be stable, complete coverage of droplet surfaces is required. If this condition is not fulfilled the dispersion is destabilized. If the dispersed droplets are coated partially with polymeric emulsifiers and the molecular weight of polymers is large enough the polymeric emulsifiers can bind up the two droplets coated with polymeric emulsifiers, i.e. polymeric emulsifiers make a bridge and cause flocculation. But at high shear rate the large flocculated droplets are disentangled and dispersed in equal distance. If the distance between droplets becomes close to any value the dispersion system shows a rapid increase in viscosity and at this time the viscoelastic behavior of the system is dependent on the ratio of $\delta$ to R. The reduction in $\delta$ with increasing volume or weight fraction of the dispersed phase ($\Phi$) may be attributed to the interpenetration and/or compression of chains with increasing $\Phi$. Also the reduction in $\delta$ value can also be attributed to compression of chains on close approach without the need to invoke any interpenetration.

Thus if the droplet size is small not only aggregation or coalescence but also precipitation is prevented. Polymeric emulsifiers give steric stability and help active components transport into the skin effectively because of the favorable absorption properties. But these properties are the main reasons that nanoemulsions can not be prepared by the conventional method of using a high pressure homogenizer (ca 500-1600 atmospheric pressure) in the condition of o/w nanoemulsions having more than 20% of inner oil phase in general. If the distance between droplets becomes less than the critical distance by high pressure the droplets become aggregated easily and heterogeneous. Therefore, novel nanoemulsification methods other than that using a high pressure homogenizer is required for preparing nanoemulsions of higher quality.

Basically steric stabilization is dependent on the flocculation of sterically stabilized dispersion. $\Theta$-point is the temperature when interaction between polymers and solvent is just as high as that between segments of the polymers. But in this invention $\Theta$-point is defined as the temperature when a viscoelastic complex of high viscosity or hardness is formed by the interaction of oil phase and water phase.

The $\Theta$-point is determined by the measurement of the viscoelastic property of emulsion systems on heating and stirring slowly. In order to prevent the coupling of the formed droplets an energy barrier should be formed. When a stabilizing agent is added to the emulsion systems an energy barrier is formed wherein, a boundary of mono molecules or multi-layer liquid crystals are formed. For the systems to be sterically stable interaction between droplets should be large enough. For these reasons good polymeric emulsifiers should have tails dissolved in the outer phase and segments insoluble in the outer phase and also segments adhered to the droplets in the inner phase, contributing to the stability of the emulsion system.

When linear polymers are put into solvent molecules they penetrate into the vacant area formed by the decreased mutual interaction between polymer molecules and the non-crystal areas first and gradually penetrate into crystal areas of high density. Therefore, in the beginning, parts of the linear polymers become swollen by coupling with the crystal area or by entanglement. Dissolution is obtained when this coupling is decoupled and molecular segments are free to move. When polymer ends are combined to any other than ends the reaction is called a cross linking reaction and the branched part is called a crosslink. If some parts of molecular chains are bound by crosslinking, each molecular chain is not separated and can not be swollen to a constant size. The crosslinking reaction occurs by the proper monomers or occurs by several reactions after polymerization. The crosslinking reactions make crosspolymers of different degrees of crosslinking by the crosslinking method and conditions applied. Crosslinking polymers of low degrees or high degrees can be obtained by controlling the number of crosslinks in the definite length of chains, fewer crosslinked polymers have a good recovering (elastic) property as described above. In any swollen state with remnant network structures by the cross-links, liquid crystal phase or entanglement is called gel. Gel is divided into viscoelastic gels and non viscoelastic gels.

Therefore, the inventors took the facts described above and studied them to improve the invention of patent (Korea Patent No.488220) of nanoemulsion cosmetic compositions containing polymeric emulsifier POP-POE vitamin E forming nanoemulsions of a mean droplet diameter of 206-455 nm and their preparation method. Finally, the inventors completed the invention of nanoemulsion cosmetic compositions with a mean droplet diameter of 43-96 nm and their preparation method by using polyacrylic acid or polyacrylic acid derivative crosspolymers as assistant emulsifiers which themselves have poor emulsifying abilities but good gel forming properties along with emulsifiers, PO-POE vitamin E, at the $\Theta$-point above 40°C, preferably above 50°C through the study of energy transferring systems of high efficiency which can absorb the rotational frictional energy by high-speed stirring.

The nanoemulsions prepared with emulsifiers, POP-POE vitamin E, along with emulsion assistants, polyacrylic acid or polyacrylic acid derivative crosspolymers by high-speed stirring in this invention showed much improved properties in the formation of viscoelastic complex of high viscosity or hardness of oil soluble phase and water soluble phase, i.e.

improved emulsification properties than those prepared only with emulsifiers, POP-POE vitamin E only due to improved properties to absorb rotational frictional energy necessary for emulsification to fine droplets.

The o/w nanoemulsion cosmetic compositions in this invention comprise 0.5 to 60% by weight, preferably 1.0 to 30 % by weight. If the content of POP-POE vitamin E is below 0.5% the stability of nanoemulsion is deteriorated and if the content of POP-POE vitamin E is over 60% the properties of nanoemulsion such as the feeling when applied on the skin become undesirable.

When POP-POE vitamin E is used as an emulsifier, the emulsifiers are adsorbed easily at the interface of oil soluble phase and water soluble phase by the easy formation of liquid crystals, resulting in the favorable formation of complex of oil soluble phase and water soluble phase, which makes smaller oil-soluble phase droplets by stirring.

And o/w nanoemusion cosmetic compositions in this invention are safe by the use of polymeric emulsifiers which are limited in penetration into the skin. In this invention the skin protecting agents and skin treating agents in the o/w nanoemulsion cosmetic compositions show more improved effects of skin protection and skin treating than in emulsion compositions by the properties of nanoemulsion such as stability and excellent skin penetration activity.

The o/w nanoemulsion cosmetic compositions in this invention comprise 0.01 to 40 % by weight emulsion assistants, polyacrylic acid or its derivative crosspolymers, preferably 0.02 to 10% by weight. It is not desirable to use polyacrylic acid or its derivative crosspolymers below 0.01% by weight since the effect of droplet size decrease is poor because of the reduction of the cohesive energy of the complex of oil soluble phase and water soluble phase. Also it is not desirable to use assistants, polyacrylic acid or its derivative crosspolymers over 40 % by weight due to an undesirable feeling when applied on the skin.

In this invention components of o/w nanomemulsion cosmetic compositions together with emulsifiers, POP-POE vitamin E emulsifiers, and emulsion assistants, polyacrylic acid or its derivative crosspolymers oil-soluble components and water soluble components being generally used to form product and to protect skin in cosmetic compositions. The representative oil soluble materials are as follows: hydrocarbon materials of paraffin oil, squalane, squalene; synthetic triglycerides such as caprylic/capric triglyceride (Ncobee M-5) obtained from the reaction of natural material; synthetic ester oils such as cetyloctanoate, octyldodecanol (Eutanol G), isopropyl palmitate; plant oils such as jojoba oil, olive oil, safflower oil, evening primrose oil, Chinese pepper oil, sesame oil, shark oil; oil soluble vitamins such as vitamin A, E, F and their derivatives and representative water soluble materials as follows; polyols such as propylene glycol, butylene glycol, glycerine, polyethylene glycol; mucopolysaccharides such as hyaluronic acid, condroitin sulfate, glucosamine, water-soluble vitamins and their derivatives such as vitamin C and panthenol.

Nanoemulsion cosmetic compositions were prepared as follows; first emulsification was carried out at a o.4 to 0.75 weight ratio of oil soluble phase to oil soluble plus water soluble phase and finally the second emulsification was carried out after adding all remnant materials.

Weight ratio ($\Phi$) of oil soluble phase to oil soluble plus water soluble phase below 0.4 is not desirable since the $\Theta$-point above 40°C is not easily obtained in these conditions because of the weakened interaction by the increase in distance between droplets, and weight ratio ($\Phi$) over 0.75 is not desirable also since the $\Theta$-point above 40°C is not easily obtained because of the weakened interaction of droplets due to the limited solubility of emulsifier and emulsion assistants in the water soluble phase therefore, the decreased interface areas.

For stability and feel when applied on the skin in this invention other than the emulsion assistants, polyacrylic acid or its derivative crosspolymers used for decreasing emulsion droplet size, polymers such as polyvinyl pyrolidone, methylcellulose, hydroxymethylcellulose (Natrosol 250HR), and thickeners such as magnesium aluminium silicate (Veegum HV), sodium aluminium silicate (Laponite XLG) are preferably used individually or together at the contents of 0.01 to 40 % by weight, but more preferably 0.05 to 20 % by weight. It is not desirable to use thickeners out of this range because the feeling when applied on the skin and stability of nanoemulsion compositions become deteriorated in this condition.

Additives of o/w nanoemulsion cosmetic compositions in this invention are used as much as 0.1 to 30% by weight of total compositions according to the purpose of the products. If the content of additives is less than 0.1 % by weight the effect of addition of additives is very small and using additives over 30 % by weight is not desirable because the stability of compositions become deteriorated and the droplet size also becomes large.

The additives described above are as follows: ultra violet ray absorbers of benzophenone derivatives such as homomenthylsalisilates, benzophenone, 2-hydroxybenzophenone, 4-methoxybenzophenone, cinnamic acid derivatives such as ethylhexyl-p-methoxy cinnamate, octylmethoxycinamate, butylmethoxydibenzophenone; skin whitening agents such as arbutin, kojic acid, a.uvaursi extract, etc.; skin cell circulation promoting agents such as alpha-hydroxy acid, pancreatin, good skin protecting agents such as allantoin, amino acid, protein, flavonoids, milk, honey; skin protecting natural plant extracts such as extracts of angelica acutiloba, cnidium officinale, Chinese bellflower, calendula arvensis, ginseng, green tea; skin coloring dihydroxyacetone and zinc oxide having a skin soothing effect. And also as the components of skin protecting agents and skin treating agents of nanoemulsions drugs for curing skin diseases might also make use of this technology. These are, some examples: hydroquinone skin depigmentation agent and those accelerating the turnover of corneous layers such as salicylic acid, alpha-hydroxy acid, sulfur and enzyme pancreatin in acne skin

curing products and skin disease curing agent hydrocortisone, a component of adrenaline hormones. The components described above can be used without any limitations and these examples do not limit the use of any other components which can be used generally in skin protecting products and skin disease curing products.

O/w nanoemulsion cosmetic compositions and O/W nanoemulsion therapeutic compositions in this invention can be classified into cream products and lotion products. The former have no fluidity and the latter have fluidity at room temperature and these products can be used according to the added active materials as several kinds of skin protecting products and skin disease curing products such as skin care cosmetics; moisture creams and lotions, night creams, eye creams, cleansing creams and lotions, sunscreen creams and lotions, suntanning creams and lotions, skin whitening creams & lotions, anti-wrinkle creams and lotions, acne care creams and lotions and atopy care creams and lotions and dispersion type make-up cosmetics; foundation, make-up base and hair care cosmetics of creams and lotions; hair dye, hair treatment cream, hair cream and skin curing topical drugs of creams and lotions; skin depigmentation products, anti-wrinkle products, acne curing products and adrenalin hormone products.

O/w nanoemulsion cosmetic compositions in this invention are prepared characteristically only under the condition that the complex of oil soluble phase and water soluble phase becomes highly viscous or hard at θ -point above 40°C. The nanoemulsion in this invention can be prepared in an economical way due to the efficient energy transfer of the high viscosity complex of oil-soluble phase and water soluble phase along with emulsifiers and emulsion assistants at θ-point wherein, the viscosity of the complex was not decreased by heating but rather was maintained or increased.

In the preparation of o/w nanoemulsion cosmetic compositions in this invention high speed rotational mixers such as propellers, dispersers, and homogenizers generating enough frictional energy can be used for forming the high-viscosity complex of oil soluble components and water soluble components above 40°C, of Θ-point.

The invention will be described more precisely through the examples below. But these examples are only suggestions in this invention and the range in this invention is not restricted to them.

<u>Examples 1-4 and Comparative Examples 1-4: O/W nanoemulsion cosmetic base compositions</u>

According to the compositions in Table 1 oil soluble materials and emulsifiers were poured into the manufacturing tank and heated to 50°C followed by adding water soluble materials previously heated and dispersed. After confirming the occurrence of Θ-point of the mixture by slow heating and stirring using a propeller mixer the o/w nanoemulsion base compositions in Examples 1-4 were prepared by emulsifying using a high speed mixer (ca. 1,000-8,000 RPM) at 1 atmospheric pressure and cooling to 30 °C.

Comparative Examples 1-4 in Table 1 were prepared by the same method employed in Examples 1-4.

[Table 1]

| Function | Component | Examples (%) by Weight | | | | Comparative Example(%) by Weight | | | |
|---|---|---|---|---|---|---|---|---|---|
| | | 1 | 2 | 3 | 4 | 1 | 2 | 3 | 4 |
| A. Oil soluble phase | Liquid paraffin | 30 | 40 | 30 | 40 | 30 | 40 | 30 | 40 |
| | Neobee M-5 | 10 | 20 | 10 | 20 | 10 | 20 | 10 | 20 |
| | Eutanol G | 10 | 10 | 10 | 10 | 10 | 10 | 10 | 10 |
| | Preservative | Q.S. | Q.S. | Q.S. | Q.S. | Q.S. | Q.S. | Q.S. | Q.S. |
| B. Emulsifier | POP(20)-POE(50) vitamin E | 2 | 2 | 4 | 4 | 2 | 2 | 4 | 4 |
| | Butylene glycol | 3 | 3 | 3 | 3 | 3 | 3 | 3 | 3 |
| | Carbopol 941 | 0.1 | - | | - | - | - | - | - |
| | Carbopol 940 | - | 0.1 | - | - | - | - | - | - |
| C. Water soluble phase | Pemulen TR-1 | - | - | 0.1 | - | - | - | - | - |
| | Pemulen TR-2 | - | - | - | 0.1 | - | - | - | - |
| | Triethanolamine | 0.1 | 0.1 | 0.1 | 0.1 | - | - | - | - |
| | Deionized water | to 100 | to 100 | to 100 | to 100 | to 100 | to 100 | to 100 | to 100 |
| $\Phi_0$(weight): [oil soluble phase (oil soluble phase[1] water soluble phase)] | | 0.51 | 0.71 | 0.52 | 0.73 | 0.51 | 0.71 | 0.52 | 0.73 |

(continued)

| Function | Component | Examples (%) by Weight | | | | Comparative Example(%) by Weight | | | |
|---|---|---|---|---|---|---|---|---|---|
| | | 1 | 2 | 3 | 4 | 1 | 2 | 3 | 4 |
| θ-Point, °C | | 63 | 67 | 65 | 75 | 60 | 65 | 65 | 75 |

Experimental Example 1: Evaluation of nanoemulsion stability by measuring the droplet sizes and the changes of droplet sizes

The droplet sizes of o/w nanoemulsion cosmetic base compositions prepared according to Examples 1-4 and Comparative Examples 1-4 in this invention were measured by the dynamic light scattering method using the Master sizer 2000(Malvern Instrument, U.K.). The measurement of droplet sizes were carried out after diluting emulsions with deionized water to 10-20% obscuration under the following conditions and the test results arc shown in Table 2.
Measuring time: 2 min, Measuring times per second: $5 \times 10^3$, Temperature : 20°C Viscosity : 0.89 centipoise, Diffraction rate of particles: 4, Diffraction rate of dispersion phase: 1.33.
Thermodynamic stability of nanoemulsions was evaluated by measuring the droplet size of nanoemulsions two times, 1 day after preparing and 6 months after storing at 40°C.

Experimental Example 2: Emulsion stability by visual observation

Stability of o/w nanoemulsion cosmetic base compositions obtained in Examples 1-4 above was evaluated by visual observation together with droplet size measurements according to the following procedure. The test results arc shown in Table 2.

Emulsion states of compositions in Examples 1-4 and Comparative Examples 1-4 were comparatively observed both 1day after preparation and 6 months after storing at 40°C. The instabilities such as sedimentation, separation, drain, creaming and coalescence were visually observed.

Stability of emulsions is evaluated by the percentage of stable portion of the total portion and is expressed by the following equation.

$$\text{Emulsion stability}(\%)=(\text{Total portion}-\text{instable portion})/\text{total portion})\times 100\%$$

[Table 2]

| Formulation No. | Droplet size (nm) | | Droplet size Increase (%) | Emulsion stability by visual observation |
|---|---|---|---|---|
| | 1 day after preparation | 6 months after storing at 40°C: | | |
| Example 1 | 73 | 77 | 5.5 | 94 |
| Example 2 | 85 | 90 | 5.9 | 96 |
| Example 3 | 52 | 53 | 3.8 | 96 |
| Example 4 | 48 | 50 | 4.2 | 98 |
| Comparative Example 1 | 253 | 275 | 9.7 | 86 |
| Comparative Example 2 | 326 | 352 | 7.9 | 85 |
| Comparative Example 3 | 205 | 223 | 8.8 | 90 |
| Comparative Example 4 | 248 | 267 | 7.7 | 90 |

As shown in Table 2, the mean droplet diameter of o/w nanoemulsion cosmetic base compositions in Example1-4 prepared by the emulsification method of simple mixing using a propeller mixer at 1 atmospheric pressure was in the

range of 48 to 85 nm 1 day after preparation, which is a very close value to the minimum droplet diameter of nanoemulsions, 20 nm. and much smaller than those in Comparative Examples 1-4. And in the evaluation of stability by measuring the droplet size 6 months after storing at 40°C the mean droplet diameter increase (%) in Examples 1-4 in this invention was about 4.8% which is smaller than that of Comparative Examples 1-4, 8.5%.

Also in Table 2 the stability(%) of o/w nanoemulsion cosmetic base compositions in Examples 1-4 by visual observation 6 months after storing at 40°C is shown. As shown in Table 2 above the compositions in Examples 1-4 were more stable than those in Comparative Examples 1-4.

Experimental Example 3: Human patch test

In order to confirm the safety of cosmetic compositions in Examples 1-4 and those in Comparative Examples 1-4 in this invention a human patch test was carried out. Confirmations of the safety of nanoemulsions is considered important since skin irritation problems occurred often with conventional nanoemulsions because of using relalively a high amount of emulsifier phospholipids to obtain a smaller droplet. The Finn chamber human patch test for o/w nanoemulsion cosmetic base compositions of Examples 1-4 in this invention was carried out and the test results are shown in Table 3.

As shown in Table 3, o/w nanoemulsion cosmetic base compositions in Examples 1-4 were safer than those in Comparative Examples 1-4.

[Table 3]

| Composition | Irritation rate (%) (n=50) | | |
|---|---|---|---|
| | after 4hr | after 48hr | mean (%) |
| Example 1 | 0 | 0 | 0 |
| Example 2 | 0 | 0 | 0 |
| Example 3 | 0.06 | 0 | 0.03 |
| Example 4 | 0.06 | 0 | 0.03 |
| Comparative Example 1 | 0 | 0 | 0 |
| Comparative Example 2 | 0.04 | 0 | 0.02 |
| Comparative Example 3 | 0.4 | 0.06 | 0.08 |
| Comparative Example 4 | 0.1 | 0.04 | 0.07 |

Example 5 and Comparative Examples 5-1~5-5: O/W nanoemulsion cosmetic base compositions

According to the compositions in Table 4, oil soluble phase and emulsifier were poured into the manufacturing tank followed by heating them to 50°C and the dispersed water soluble phase preheated to 50°C was added. The o/w nanoemulsion cosmetic base composition in Example 5 was prepared by the emulsification method using a high speed disperser mixer (ca. 1,000-8,000 RPM) at 1 atmospheric pressure after confirming the formation of high viscosity complex at Θ-point by stirring slowly on heating the mixtures of oil soluble phase with emulsifier and water-soluble phase and cooling down to RT. Those of Comparative Examples 5-1, -2, -3, -4, -5 in Table 4 were prepared by the same method employed in Example 5.

[Table 4]

| Function | Component | Example (% by weight) | Comparative Example (% by weight) | | | | |
|---|---|---|---|---|---|---|---|
| | | 5 | 5-1 | 5-2 | 5-3 | 5-4 | 55 |
| A. Oil soluble phase | Cetostearyl alcohol | 3 | 3 | 3 | 3 | 3 | 3 |
| | Paraffin | 5 | 5 | 5 | 5 | 5 | 5 |
| | Paraffin oil | 40 | 40 | 40 | 40 | 40 | 40 |
| | Neobee M5 | 15 | 15 | 15 | 15 | 15 | 15 |
| | Eutanol G | 5 | 5 | 5 | 5 | 5 | 5 |
| | Preservative | Q.S. | Q.S. | Q.S. | Q.S. | Q.S. | Q.S. |
| B. Emulsifier | POP(20)-POE(50) vitamin E | 3 | 3 | 3 | - | - | |
| | POL(40) Vitamin E | - | - | | 3 | - | - |
| | Soybean phospholipid | - | - | - | - | 3 | - |
| | Polysorbate-60/ Sesquioleate | -/ | -/- | -/- | -/- | -/- | 1.7/ 0.3 |
| C Water soluble phase | Bulylene glycol Carbopol 941 | 3 | 3 | 3 | 3 | 3 | 3 |
| | | 0.12 | | - | 0.06 | 0.06 | 0.06 |
| | Natrosol 250HR | - | - | 0.2 | - | - | - |
| | Triethanolamine | 0.1 | - | - | 0.1 | 0.1 | 0.1 |
| | Deionized water | to 100 | to 100 | to 100 | to 100 | to 100 | to 100 |
| $\Phi_0$(weight) [Oil soluble phase / (oil soluble phase water soluble phase)] | | 0.72 | 0.72 | 0.72 | 0.72 | 0.72 | 0.72 |
| θ-Point, °C | | 70 | 72 | 68 | - | - | - |

Experimental Example 1: Revaluation of nanoemulsion by measuring the droplet sizes and the changes of droplet sizes

The droplet sizes of o/w nanoemulsion cosmetic base compositions prepared according to Example 5 and Comparative Examples 5-1 to 5-5 in this invention were measured by the same method employed in Experimental Example 1-4 in Example 1-4 and the test result 1 day after and 6 months after storing at 40 °C arc shown in Table 5.

[Table 5]

| Formulation No. | Droplet size, nm | | Droplet size Increase (%) |
|---|---|---|---|
| | 1 day after storing at RT | 6 months after storing at 40°C | |
| Example 5 | 45 | 46 | 2.2 |
| Comparative Example 5-1 | 228 | 248 | 10.0 |
| comparative Example 5-2 | 241 | 258 | 7.1 |
| Comparative Example 5-3 | | - | - |
| Comparative Example 5-4 | - | - | |
| Comparative Example 5-5 | | - | - |

The "-" sign in Table 5 means that the composition was not emulsified by the emulsification method employed in this invention.

As shown in Table 5 the mean droplet diameters of o/w nanoemulsion cosmetic base composition of Example

5 in this invention prepared by the simple rotational mixing method using a disperser mixer at 1 atmospheric pressure 1 day after preparation at room temperature(RT) was 45 nm, which is close to the minimum value of the nanoemulsion droplets diameter range, 20-500 nm but that of Comparative Examples 5-1 and 5-2 was 235 nm. On the other hand, nanoemulsions were not prepared when the base compositions of Comparative Examples 5-3. 5-4 and 5-5 were emulsified by the same nanoemulsification method using a simple rotational mixer employed in Example 5. Namely, those compositions did not form the high viscosity complex when the oil soluble phase was mixed with the water soluble phase along with an emulsifier and an emulsion assistant, Carbopol i.e. the mixture did not show a Θ-point. The mean droplet diameter of the compositions of Comparative Examples 5-3 5-4 and 5-5 were 1.675 nm, 2.278 nm and 3.258 nm, respectively, when prepared by the emulsification method using a homogenizer. Increase (%) in mean droplet diameters of composition in Example 5 by evaluating the droplet diameters of the emulsion compositions 6 months after storing at 40°C was ca. 2.2%, which is much smaller than that of Comparative Examples 5-1 and 5-2, ca.8.6%.

Examples 6-7 and Comparative Examples 6-7: O/W nanoemulsion cosmetic cream compositions

The purpose of Examples 6-7 is to confirm the effect of additives in preparing nanoemulsions since the property of nanoemulsions can be affected by the additives even though the droplet diameter of emulsion or nanoemulsion is mainly dependent on the base components.

According to the compositions in Table 1, oil soluble materials and emulsifiers were poured into the manufacturing tank and heated to 50 °C followed by adding water soluble materials previously heated to 50 °C and dispersed. After confirming the occurrence of Θ-print of the mixture by slow heating and stirring using a propeller mixer the o/w nanoemulsion base compositions in examples 1-4 were prepared by emulsifying using a high speed mixer (ca. 1,000-8,000 RPM) at 1 atmospheric pressure and cooling to 30 °C.

Comparative Examples 1-1 in Table 1 were manufactured by the same method for comparison.

According to compositions in Table 6, oil-soluble raw materials 1-11 were poured into a manufacturing tank followed by melting on heating to 7 0°C. The tank was kept after adding raw materials 12-14. Next, water soluble raw materials 18-29 dissolved in a supplementary tank at 50°C were added to the manufacturing tank. Afterwards, the high viscosity 2 phase complex of oil soluble phase and water soluble phase were prepared by slowly mixing using a homogenizer on increasing the temperature to Θ-point. O/w nanoemulsion moisture cream was prepared by cooling homogenized emulsions to 30°C, which had been subjected previously to stirring again using a disperser (1,000-8,000 RPM) after cooling to 50 °C and adding oil soluble materials 15-17 to the high viscosity complex at 1 atmospheric pressure.

[Table 6]

| Components | | Example 6 | Example 7 | Comparative Example 6 | Comparative Example 7 |
|---|---|---|---|---|---|
| | | 6 | 7 | 6 | 7 |
| 1 | Microcrystalline wax | 3.0 | 3.0 | 3.0 | 3.0 |
| 2 | Paraffin | 2.0 | 2.0 | 2.0 | 2.0 |
| 3 | Bees wax | 3.0 | 3.0 | 3.0 | 3.0 |
| 4 | Cetostearyl alcohol | 2.0 | 2.0 | 2.0 | 2.0 |
| 5 | Glycerylmonostearate | 2.0 | 2.0 | 2.0 | 2.0 |
| 6 | Liquid paraffin | 25.0 | 25.0 | 25.0 | 25.0 |
| 7 | Neobee M5 | 18.0 | 18.0 | 18.0 | 18.0 |
| 8 | Eutanol G | 5.0 | 5.0 | 5.0 | 5.0 |
| 9 | Evening primrose oil | 0.5 | 0.5 | 0.5 | 0.5 |
| 10 | Preservative | Q.S. | Q.S. | Q.5. | Q.S. |
| 11 | Antioxidant | Q.S. | Q.S. | Q.S. | Q.S. |
| 12 | POP(30)-POE(70) vitamin E | 3.0 | 2.5 | 3.0 | 2.5 |
| 13 | POP(10)-POE(30) vitamin E | 0.5 | 0.5 | 0.5 | 0.5 |

(continued)

| Components | | Example 6 | Example 7 | Comparative Example 6 | Comparative Example 7 |
|---|---|---|---|---|---|
| | | 6 | 7 | 6 | 7 |
| 14 | POP(70)-POE(150) vitamin E | 0.5 | 0.5 | 0.5 | 0.5 |
| 15 | Vitamin A palmitate | 0.2 | 0.2 | 0.2 | 0.2 |
| 16 | Vitamin E acetate | 0.5 | 0.5 | 0.5 | 0.5 |
| 17 | Perfume | Q.S. | Q.S. | Q.S. | Q.S. |
| 18 | Glycerine | 6.0 | 6.0 | 6.0 | 6.0 |
| 19 | Butylene glycol | 4.0 | 4.0 | 4.0 | 4.0 |
| 20 | Allantoin | 0.1 | 0.1 | 0.1 | 0.1 |
| 21 | Hyaluronic acid | 1.0 | 1.0 | 1.0 | 1.0 |
| 22 | Sequestering agent | Q.S. | Q.S. | Q.S. | Q.S. |
| 23 | Bearberry extract | 1.0 | 1.0 | 1.0 | 1.0 |
| 24 | Angelica actutiloba extract | 5.0 | 5.0 | 5.0 | 5.0 |
| 25 | Cnidium officinale extract | 3.0 | 3.0 | 3.0 | 3.0 |
| 26 | Carbopol 940 | 0.1 | 0.06 | - | - |
| 27 | Natrosol 250HR | - | - | 0.2 | - |
| 28 | Triethanolamine | 0.1 | 0.1 | - | - |
| 29 | Deionized water | to 100 | to 100 | to 100 | to 100 |
| $\Phi_0$(Weight) [Oil soluble phase / (Oil soluble phase + Water soluble phase)] | | 0.63 | 0.62 | 0.63 | 0.62 |
| θ-Point, °C. | | 68 | 72 | 70 | 72 |

Experimental Example 1: Evaluation of nanoemulsion stability by measuring the droplet size and the change of droplet size

For evaluating nanoemulsion stability of o/w nanoemulsion cosmetic moisture cream compositions obtained in Examples 6-7 and Comparative Examples 6-7 the mean droplet sizes of those compositions were measured by the same method employed in Experimental Example 1 of Examples 1-4.

The test result of the mean droplet, diameters of o/w nanoemulsion moisture cream compositions of Examples 6-7 and Comparative Examples 6-7 in Table 6, 1 day after preparation and 6 months after storing at 40 °C are shown in Table 7.

[Table 7]

| Formulation No. | Droplet size, nm | | Droplet size increase (%) |
|---|---|---|---|
| | 1 day after storing preparation | 6 months after storing at 40°C | |
| Example 6 | 48 | 46 | 4.3 |
| Example 7 | 51 | 50 | 0.0 |
| Comparative Example 6 | 231 | 241 | 4.3 |
| Comparative Example 7 | 215 | 230 | 7.0 |

As shown in Table 7 the mean droplet diameter of o/w nanoemulsion cosmetic compositions prepared by simple rotational mixing using a disperser mixer 1 day after preparation was in the range of 48 to 51 nm, which is a very close value to the minimum value of nanoemulsions, 20 nm.

On the other hand the mean droplet diameter of nanoemulsion compositions of Comparative Examples 6-7 was in the range of 215 to 231 nm, which is larger than that of Examples 6-7 by 182 nm. In the evaluation of stability by measuring the mean droplet diameter after storing for 6 months at 40°C the increase (%) of the mean droplet diameter of compositions of Examples 6-7 in this invention was 1.1%, which is much smaller than that of Comparative Examples 6-7, 5.7%.

Experimental Example 2: Observation of nanoemulsions by Cryogenic-Transmission Electron Microscope (Cryo-TEM)

Droplet sizes of emulsions in general can be measured by the indirect method of dynamic light scattering using a laser light. But in case of smaller nanoemulsions a direct measuring method along with an indirect measuring method provides more precise droplet size data for nanoemlsions. Observing fine droplets using a Cryo-TEM is a direct precise method, which can measure the droplet size without changing the state. Therefore, Cryo-TEM was used to confirm the mean droplet diameter of o/w nanoemulsion of Example 6, 45 nm, by the light scattering method, which is close to the minimum value of nanoemulsions, 20 nm. Cryo-TEM (CEM902A. Zeiss, I)-Oberkochen, Philips, CM120, NL-Edinhoven) was operated both at 80 kV and 120 kV. An image of nanoemulsion droplets was acquired for the nanoemulsions diluted with deionized water, sonicated and applicated on a copper grid as thin as possible and frozen at 77-100 °K for image treating systems (Kontron IBAS).

Figure 1 is a microphotograph of Cryo-TEM for the nanoemulsion composition of Example 6, 1 day after preparation. From Figure 1 we can see that the mean droplet diameter of o/w nanoemulsion cosmetic moisture cream of Example 6 is 50 nm, which is very similar to the value of 48 nm obtained by the dynamic light scattering method.

Figure 2 is a microphotograph of nanoemulsions in Comparative Example 6, 1 day after preparation. From Figure 2 we can see that the mean droplet diameter of nanoemulsion compositions of Comparative Example 6 is 210 nm, which is also a very similar value, to 231 nm that obtained by the dynamic light scattering method.

Experimental Example 3: Observation of emulsion droplets by atomic force microscope (AFM)

Recently, atomic force microscopes (AFM) are used for the morphological study of materials. The merit of using this device is that the particles can be observed at RT and at 1 atmospheric pressure without any deformation of particles. In Experimental Example 3 an AFM was employed to observe the droplets of o/w nanoemulsion cosmetic moisture cream in Example 7 in this invention.

The test sample was prepared using a pyramidal silicon nitride contilever with a force constant at 0.26 N/m. A photographic image was obtained with a non-contact scanning probe microscope (AutoProbe-CP: Park Scientific Instruments, CA, U.S.A.).

Figure 3 is an AFM mocrophotograph of nanoemulsion cosmetic moisture cream in Example 7 in this invention 1 day after preparation and Figure 4 is that in Comparative Example 7, 1 day after preparation.

The mean droplet diameters of nanoemulsions in Figure 3 and Figure 4 are 43 nm and 186 nm, respectively.

Experimental Example 4: Product Effectiveness Test

To measure the effectiveness of o/w nanoemulsion cosmetic moisture cream compositions in Example 6 and Comparative Example 6, 4 groups of 80 women aged 20 to 50 were employed for a 3 month test. Among them 30 women had a atopy skin bothered with severe dryness and itching. The main items of use in the test were the effects of moisturizing, skin firming, skin wrinkle decreasing, whitening and atopy skin soothing, which were observed in priority. Other effects such as stickiness during application, spreadability on the skin, skin wetness and skin sheen were evaluated and given 4 grades of excellent, good, average and poor. The test results are shown in Table 8.

[Table 8]

| Item/Product | Example 6 | Comparative Example 6 |
|---|---|---|
| Wrinkle decreasing effect | OOO | OO |
| Whitening effect | OO | OO |
| Spreadability on skin | OOO | OO |
| Atopy skin soothing effect | OOO | OO |

(continued)

| Item/Product | Example 6 | Comparative Example 6 |
|---|---|---|
| Smoothness | OOO | OO |
| moisturizing effect | OOO | OO |
| Skin sheen | OOO | OOO |
| OOO: Excellent. OO: Good, O: Average, x: Poor | | |

As shown in Table 8 o/w nanoemulsion cosmetic moisture cream in Example 6 in this invention showed better effects than that in Comparative Example 6 and particularly, an improved effect on itchy atopy skin caused from severe dryness was excellent.

Examples 8-9 and Comparative Examples 8-9: o/w nanoemulsion cosmetic moisture lotion compositions

So far the inventors have described cream o/w nanoemulsions cosmetic compositions and their preparation method through Examples 1-7 and Comparative Examples 1-7. Now the inventors will explain lotion or nanoemulsion lotion. It is generally defined as cream with no fluidity but lotion with fluidity at RT.

O/w lotions have in general less amount of waxes with high melting points and oils as inner phase than creams and have a characteristic soft feel when applied on the skin. Emulsion lotions are stabilized using polymeric thickeners such as Carbomers since the thermal stability of emulsion lotions is much inferior to that of creams due to a lower viscosity. But these polymers do not generally make small emulsion droplets. Basically emulsion lotions have very similar compositions to that of emulsion creams except for having a much smaller amount of wax with a high melting point and oil soluble components. Therefore, emulsion lotions have very similar effects to those of cream.

According to the compositions in Table 9, oil soluble components, raw materials 1-11, were poured into a manufacturing tank and melted on heating to 70°C. Subsequently, emulsifiers, raw materials 12-14, were added and dissolved in the tank and was maintained at 50°C. Next, water soluble components, raw materials 17-26, dissolved previously in a supplementary tank at 50°C were added to the manufacturing tank. Then, the high viscosity 2-phase complex of oil soluble phase and water soluble phase was formed by slowly mixing using a propeller mixer (1,000-8,000RPM) on increasing temperature to Θ-point. Homogeneous emulsion compositions were prepared by high speed mixing of the previous high viscosity complex using a propeller mixer at 1 atmospheric pressure and cooling to 50°C. And next, raw material 27 was added to the homogeneous emulsion composition, which was stirred again at 1,000-8,000 RPM. Finally, o/w nanoemulsion cosmetic moisture lotion was prepared by stirring the homogeneous emulsion composition homogeneously after adding oil soluble components, raw materials 15-16 and cooling to 30°C. Compositions of Comparative Examples 8-9 were prepared by the same method employed in those of Examples 8-9.

[Table 9]

| Component | | Example | | Comparative Example | |
|---|---|---|---|---|---|
| | | 8 | 9 | 8 | 9 |
| 1 | Microcrystalline wax | 1.0 | 1.0 | 1.0 | 1.0 |
| 2 | Vaseline | 0.5 | 0.5 | 0.5 | 05 |
| 3 | Bees wax | 0.5 | 0.5 | 0.5 | 0.5 |
| 4 | Cetostearyl alcohol | 2.0 | 2.0 | 2.0 | 2.0 |
| 3 | Glyceryl monostearate | 1.2 | 1.2 | 1.2 | 1.2 |
| 6 | Liquid paraffin | 22.0 | 22.0 | 22.0 | 22.0 |
| 7 | Neobee M-5 | 8.0 | 8.0 | 8.0 | 8.0 |
| 8 | Eutanol G | 5.0 | 5.0 | 5.0 | 5.0 |
| 9 | Shear butter | 0.5 | 0.5 | 0.5 | 0.5 |
| 10 | Preservative | Q.S. | Q.S. | Q.S. | Q.S. |
| 11 | Antioxidant | Q.S. | Q.S. | Q.S. | Q.S. |
| 12 | POP(20)-POE(50) vitamin E | 2.5 | 2.0 | 2.5 | 2.5 |

(continued)

| Component | | Example | | Comparative Example | |
|---|---|---|---|---|---|
| | | 8 | 9 | 8 | 9 |
| 13 | POP(10)-POE(30) vitamin R | 0.5 | 0.5 | 0.5 | 0.5 |
| 14 | POP(100)-POE(20) vitamin E | 0.3 | 0.3 | 0.3 | 0.3 |
| 15 | Vitamin E Acetate | 0.3 | 0.3 | 0.3 | 0.3 |
| 16 | Perfume | Q.S. | Q.S. | Q.S. | Q.S. |
| 17 | Butylene glycol | 6.0 | 6.0 | 6.0 | 6.0 |
| 18 | Allantoin | 0.1 | 0.1 | 0.1 | 0.1 |
| 19 | Ilyaluronic acid | 1.0 | 1.0 | 1.0 | 1.0 |
| 20 | Betaglucan | 0.1 | 0.1 | 0.1 | 0.1 |
| 21 | Rosemary extract | 5.0 | 5.0 | 5.0 | 5.0 |
| 22 | Cnidium officinale extract | 5.0 | 5.0 | 5.0 | 5.0 |
| 23 | Carbopol 934 | 0.1 | - | - | - |
| | Pemulen TR 2 | - | 0.1 | - | - |
| 24 | Natrosol 250 HR | - | - | 0.1 | - |
| 25 | Triethanolamine | 0.1 | 0.1 | - | - |
| 26 | Deionized water-1 | 25 | 25 | 25 | 25 |
| 27 | Deionized water-2 | to 100 | to 100 | to 100 | to 100 |
| | $?_{01}$ (Weight)/$?_{02}$ (Weight) [Oil soluble phase/ (Oil soluble phase + Water soluble phase)] | 0.62 /0.42 | 0.62 /0.42 | 0.62 /0.42 | 0.62 /0.42 |
| | $\theta_1$-Point, °C | 71 | 72 | 70 | 71 |

Experimental Example 1: Evaluation of nanoemulsion stability by measuring droplet sizes and the change of droplet sizes

For confirming droplet sizes and evaluating the stability of o/w nanoemulsion cosmetic moisture lotion compositions, the droplet sizes of compositions in Examples 8-9 and Compositions Examples 8-9 were measured by the same method employed in Experimental Example 1 in Examples 1-4. The emulsion droplet diameter test results of o/w nanoemulsion moisture lotion compositions in Examples 8-9 and those in Comparative Examples 8-9 in Table 9,1 day after preparation and 6 months after storing at 40°C are shown in Table 10.

[Table 10]

| Formulation No. | Droplet size, nm | | Droplet size increase (%) |
|---|---|---|---|
| | 1 day after storing preparation | 6 months after storing at 40°C | |
| Example 8 | 85 | 88 | 3.5 |
| Example 9 | 87 | 92 | 5.7 |
| Comparative Example 8 | 316 | 336 | 8.2 |
| Comparative Example 9 | 350 | 382 | 9.1 |

As shown in Table 10, the mean droplet diameters of nanoemulsion compositions in Examples 8-9 prepared by simple mixing using a propeller stirrer 1 day after and 6 month after storing at 40°C in this experiment were below 100 nm and the increase(%) in emulsion droplet sizes 6 months after storing at 40°C had a relatively low value of 4.6%. On the other hand, the mean droplet diameter of nanoemulsion compositions prepared in Comparative Examples 8-9 was 333 nm, which is also in the droplet size range of nanoemulsions but much larger than that of compositions in

Examples 8-9.

Examples 10-11 and Comparative Examples 10-11: O/W nanoemulsion skin depigmentation cream compositions

According to compositions in Table 11, oil soluble components, raw materials 1-9, were poured into a manufacturing tank and melted on heating to 70°C. Subsequently, emulsifiers, raw materials 10-11, were added and dissolved in the tank and the tank was maintained at 50°C. Next, water soluble components, raw materials 12-19 and 22, dissolved previously in a supplementary tank at 50°C was added to the manufacturing tank. Then, the 2 phase complex of high viscosity of oil soluble phase and water soluble phase was formed by slowly mixing using a disperser. Next, homogeneous emulsion compositions were prepared by high speed mixing of the previous complex using a disperser (1,000-8,000 RPM) at 1 atmospheric pressure and cooling to 50°C. And next, raw material 20 previously dissolved in deionized water was added to the homogeneous emulsion composition. Finally, o/w nanoemulsion cosmetic moisture lotion was prepared by stirring the above emulsion composition homogeneously using a disperser followed by adding raw material 21 to adjust the pH to 4.0 to 6.0 and cooling to 30 °C. Compositions in Comparative Examples 10-11 were prepared by the same method employed in those in Examples 10-11.

[Table 11]

| Component | | Example | | Comparative Example | |
|---|---|---|---|---|---|
| | | 10 | 11 | 10 | 11 |
| 1 | Bees wax | 4.0 | 4.0 | 4.0 | 4.0 |
| 2 | Paraffin was | 3.0 | 3.0 | 3.0 | 3.0 |
| 3 | Cetostearyl alcohol | 2.0 | 2.0 | 2.0 | 2.0 |
| 4 | Glyceryl monostearate | 1.0 | 1.0 | 1.0 | 1.0 |
| 5 | Liquid paraffin | 25.0 | 25.0 | 25.0 | 25.0 |
| 6 | Squalane | 20.0 | 20.0 | 20.0 | 20.0 |
| 7 | Dimethylnolysiloxane | 0.5 | 0.5 | 0.5 | 0.5 |
| 8 | Preservative | Q.S. | Q.S. | Q.S. | Q.S. |
| 9 | Antioxidant | Q.S. | Q.S. | Q.S. | Q.S. |
| 10 | POP(25)-POE(60) vitamin E | 2.5 | 3.0 | 2.5 | 3.0 |
| 11 | POP(5)-POE(0) vitamin E | 0.5 | 0.5 | 0.5 | 0.5 |
| 12 | Carbopol 934 | 0.4 | 0.06 | - | - |
| 13 | Voegum HV | - | 0.2 | - | 0.2 |
| 14 | Sodium metabisulfite | 0.2 | 0.2 | 0.2 | 0.2 |
| 15 | Sorbic acid | 0.2 | 0.2 | 0.2 | 0.2 |
| 16 | Glycerin | 7.0 | 7.0 | 7.0 | 7.0 |
| 17 | Propylene glycol | 5.0 | 5.0 | 5.0 | 5.0 |
| 18 | Triethanolamine | 0.1 | | - | - |
| 19 | EDTA-2Na | 0.1 | 0.1 | 0.1 | 0.1 |
| 20 | Hydroquinone | 4.0 | 4.0 | 4.0 | 4.0 |
| 21 | Citric acid | Q.S. | Q.S. | Q.S. | Q.S. |
| 22 | Deionized wate | to 100 | to 100 | to 100 | to 100 |
| $\phi_0$(Weight) [Oil soluble phase / (Oil soluble phase + Water soluble phase)] | | 0.57 | 0.58 | 0.57 | 0.58 |
| $\theta$-Point. °C | | 72 | 73 | 72 | 76 |

Experimental Example 1: Evaluation of nanoemulsion stability by measuring emulsion droplet sizes and the change of droplet sizes

For confirming droplet sizes and evaluating the stability of o/w nanoemulsion skin depigmentation cream compositions in Examples 10-11 the droplet sizes of compositions in Examples 10-11 and those in Comparative Examples 10-11 were measured by the same method employed in Experimental Example 1 of Examples 1-4.
In Table 12, the test results of droplet sizes of compositions in Examples 10-11 and Comparative Examples 10-11 in Table 11 are shown.

[Table 12]

| Formulation No. | Droplet size, nm | | Droplet size increase (%) |
|---|---|---|---|
| | 1 day after storing preparation | 6 months after storing at 40 °C | |
| Example 10 | 65 | 68 | 4.6 |
| Example 11 | 72 | 71 | 0.0 |
| Comparative Example 10 | 313 | 335 | 7.0 |
| Comparative Examples 11 | 257 | 278 | 8.1 |

As shown in Table 12, the droplet diameter of o/w nanoemulsion cosmetic skin depigmentation cream compositions in Examples 10-11 is 69 nm, which is a very close value to the minimum diameter of nanoemulsions, 50 nm.
On the other hand, the mean droplet diameter of compositions in Comparative Examples 10-11 is 307 nm, which is much larger than that of compositions in Examples 10-11. And the mean droplet size increase(%) of nanoemulsions in Examples 10-11 is 2.3 % and that of Comparative Examples 10-11 is 7.0 %, respectively.

Examples 12-13 and Comparative Examples 12-13: O/w nanoemulsion acne skin treating cream compositions

According to the compositions in Table 13, oil soluble raw materials 1-9 and emulsifiers, raw materials 10-14, were poured into a manufacturing tank, which was heated to 70°C. Then, a high viscosity complex of an oil soluble phase and a water soluble phase was prepared by slowly mixing using a homogenizer after adding the water soluble raw materials 17-22 previously dispersed homogeneously in a complementary tank. Next, o/w nanoemulsion acne skin treating cream was prepared by stirring the mixture of the previous homogeneous complex using a propeller mixer after adding raw materials 15-16 and cooling to 30°C.

[Table 13]

| Component | | Example | | Comparative Example | |
|---|---|---|---|---|---|
| | | 12 | 13 | 12 | 13 |
| 1 | Paraffin | 3.0 | 3.0 | 3.0 | 3.0 |
| 2 | Bees wax | 3.5 | 3.5 | 3.5 | 3.5 |
| 3 | Shear butter | 1.0 | 1.0 | 1.0 | 1.0 |
| 4 | Glyceryl monostearate | 1.3 | 1.3 | 1.3 | 1.3 |
| 5 | Liquid paraffin | 25.0 | 25.0 | 25.0 | 25.0 |
| 6 | Squalane | 15.0 | 15.0 | 15.0 | 15.0 |
| 7 | Eutanol G | 5.0 | 5.0 | 5.0 | 5.0 |
| 8 | Phenoxythanol | 0.2 | 0.2 | 0.2 | 0.2 |
| 9 | Polydimethylsiloxane | 0.5 | 0.5 | 0.5 | 0.5 |
| 10 | POP(20)-POE(50) vitamin E | 2.5 | 3.5 | 2.5 | 3.5 |
| 11 | POP(100)-POE(200) vitamin E | 0.5 | 0.5 | 0.5 | 0.5 |
| 12 | Perfume | Q.S. | Q.S. | Q.S. | Q.S. |

(continued)

| Component | | Example | | Comparative Example | |
|---|---|---|---|---|---|
| | | 12 | 13 | 12 | 13 |
| 13 | Sorbic acid | 0.2 | 0.2 | 0.2 | 0.2 |
| 14 | Sulfur | 0.5 | 0.5 | 0.5 | 0.5 |
| 15 | Acritamer 941 | 0.1 | 0.15 | - | - |
| 16 | Triethanolamine | 0.1 | - | - | - |
| 17 | Natrosol 250HR | - | - | 0.2 | 0.2 |
| 18 | Butylene glycol | 7.0 | 7.0 | 7.0 | 7.0 |
| 19 | Zinc oxide | 2.0 | 2.0 | 2.0 | 2.0 |
| 20 | Sodium metasulfate | 0.1 | 0.1 | 0.1 | 0.1 |
| 21 | Lactic acid | 0.5 | 0.5 | 0.5 | 0.5 |
| 22 | Deionized water | tu 100 | to 100 | to 100 | to 100 |
| $\phi_0$(Weight) [Oil soluble phase / (Oil soluble phase + Water soluble phase)] | | 0.56 | 0.67 | 0.56 | 0.57 |
| θ-Point, °C | | 75 | 72 | 76 | 75 |

<u>Experimental Example 1: Evaluation of nanoemulsion stability by measuring emulsion droplet sizes and the chance of droplet sizes</u>

For confirming droplet sizes and evaluating the stability of o/w nanoemulsion acne skin treating cream compositions, droplet sizes of compositions in Examples 12-13 and those in Comparative Examples 12-13 were measured by the same method employed in Experimental Example 1 of Example 1-4.

The test results of droplet size measurement of o/w nanoemulsion cosmetic acne skin treating cream compositions I day after preparation and 6 months after storing at 40°C are shown in Table 14.

[Table 14]

| Formulation No. | Droplet size, nm | | Droplet size increase (%) |
|---|---|---|---|
| | 1 day after storing preparation | 6 months after storing at 40°C | |
| Example 12 | 95 | 102 | 7.4 |
| Example 13 | 89 | 96 | 6.3 |
| Comparative Example 12 | 427 | 486 | 13.8 |
| Comparative Example 13 | 459 | 503 | 9.5 |

As shown in Table 14 the mean droplet diameter of o/w nanoemulsion acne skin treating cream compositions in Example 12-13 is 92 nm, which is close to the minimum value of nanoemulsion droplet size range of 20 to 50 nm in this invention. On the other hand that of Comparative Examples 12-13 is 443 nm, which is close to the maximum value of nanoemulsion droplet size, 500 nm.

The mean droplet size increase(%) of nanoemulsions in Examples 12-13. 6 months after storing at 40°C is 6.9% but that of Comparative Examples 12-13, 6 months after storing at 40°C is 11.7%.

[Industrial Applicability]

In this invention nanoemulsions containing POP-POE vitamin E as emulsifiers and polyacrylic acid or polyacrylic acid derivative crosspolymers as emulsion assistants have a very small mean droplet diameter ranging from 43 to 96 nm and are very stable and safe.

By the nanoemulsions described above the inventors obtained nanoemulsion cosmetic compositions with a

much smaller mean droplet diameter than nanoemulsions prepared without emulsion assistants, polyacrylic acid or polyacrylic acid derivative crosspolymers by emulsifying using a simple high speed mixer such as a propeller, a disperser and a homogenizer due to the much improved properties when forming viscoelastic complex of oil soluble phase and water soluble phase above 40°C.

The economical productivity of the invention is very high since the nanoemulsion cosmetic compositions could be prepared by simple mixing at 1 atmospheric pressure in this invention. Also nanoemulsion cosmetic compositions in this invention have good effects such as skin absorption, skin softening, skin moisturizing, skin firming, improving itchy dry skin and good spreadability when applied on the skin and particularly, have the merits of a long lasting effect due to the favorable penetration of active drug components into the skin.

## Claims

1. A nanoemulsion comprising polyoxypropylene-polyoxyethylene vitamin E represented by the following general formula 1 as an emulsifier and polyacrylic acid or polyacrylic acid derivative crosspolymer as an emulsion assistant:

[General formula 1]

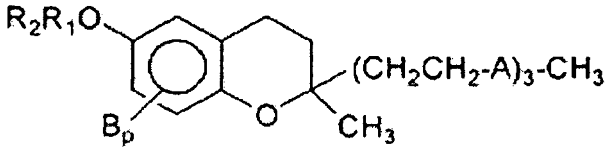

(1)

wherein, $R_1$ is $-(O-CH_2CH_2)_m-$ m is an integer of 0 to 300, $R_2$ is $H(OCH(CH_3)CH_2)_n-$, n is an integer of 1 to 250; A is

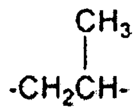

or $-C(CH_3)=CH-$; B is $-CH_3$ at the 5, 7 or 8 position of vitamin E; and p is an integer of 1 to 3.

2. The nanoemulsion according to claim 1, wherein the polyacrylic acid or polyacrylic acid derivative crosspolymers is a Carbomer, a homopolymer of acrylic acid crosslinked with an allyl ether of pentaerythritol, an allyl ether of sucrose or an allyl ether of propylene or acrylates/$C_6$-$C_{40}$ alkyl acrylate crosspolymers, copolymer of $C_6$-$C_{40}$ alkyl acrylates and one or more nanomers of acrylic acid, methacrylic acid or one of their simple esters crosslinked with an allyl ether of sucrose or an allyl ether of penta erythritol represented by the following general formula 2:

[Genenal formula 2]

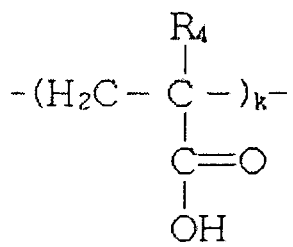

wherein, $R_4$ is H or $CH_3$, k is an integer of 10 to 100,000.

3. The nanoemulsion according to claim 2, wherein the ether of polyol is an allyl ether of pentaerythritol or an allyl ether of sucrose.

4. The nanoemulsion according to claim 1, wherein the vitamin E of polyoxypropylene-polyoxyethylene vitamin E is a natural vitamin E or a synthetic vitamin E.

**5.** The nanoemulsion according to claim 1, wherein the content of polyoxypropylene-polyoxyethylene vitamin E in the total nanoemulsion is 0.5 to 60% by weight.

**6.** The nanoemulsion according to claim 2, wherein the content of the polyacrylic acid or the polyacrylic acid derivative crosspolymer of total nanoemulsion is 0.01 to 40% by weight.

**7.** The nanoemulsion according to claim 1, wherein mean droplet diameter of the nanoemulsion is in the range of 43 to 96 nm.

**8.** A cosmetic non-therapeutic composition comprising the nanoemulsion according to claim 1 to claim 7.

**9.** The cosmetic composition according to claim 8, wherein the cosmetic composition is one product selected among the group of cream or lotion type products of skin protecting, atopy skin improving, skin depigmentation and wrinkle improving.

**10.** A composition comprising a nanoemulsion according to any of claim 1 to claim 7 for use in a therapeutic treatment for treating the skin selected from acne treating.

**11.** A composition for the use according to claim 10 wherein the composition is one product selected among the group of cream or lotion type products of an acne treating and adrenaline cortex hormone.

**12.** A preparation method of a nanoemulsion cosmetic composition, wherein oil soluble components and water soluble components are mixed with polyoxypropylene-polyoxyethylene viatmin E expressed in general formula 1 as emulsifier and polyacrylic acid or polyacrylic acid derivative crosspolymer as emulsion assistant:

[General formula 1]

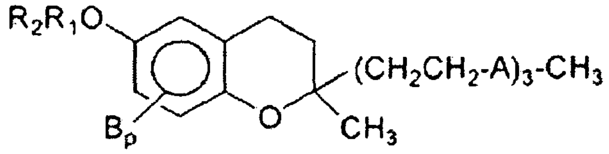

(1)

wherein, $R_1$ is $-(O-CH_2CH_2)_m-$, m is an integer of 0 to 300, $R_2$ is $H(OCH(CH_3)CH_2)_n-$, n is an integer of 1 to 250; A is

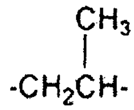

or- $C(CH_3)=CH-$; B is $-CH_3$ at the 5-, 7- or 8- position of vitamin E; and p is an integer of 1 to 3.

**13.** The preparation method according to claim 12, wherein the nanoemulsion cosmetic composition is prepared by emulsifying the viscoelastic complex of oil soluble phase and water soluble phase, wherein the weight ratio($\phi$ ) of oil soluble phase to oil soluble phase plus water soluble phase is 0.4 to 0.75, and then if necessary emulsifying the emulsified viscoelastic complex again after adding the remnant oil soluble phase and water soluble phase.

**14.** The preparation method according to claim 12 or claim 13, wherein the nanoemulsion cosmetic composition is prepared by high speed mixing of the complex of oil soluble phase and water soluble phase at $\theta$ -point above 40°C when the previous complex shows viscoelastic properties.

**15.** The preparation method according to claim 14, wherein the nanoemulsion cosmetic composition is prepared at 1 atmospheric pressure by a high speed mixer selected from a group comprised of a propeller, a disperser, an agitator and a homogenizer.

**Patentansprüche**

1. Nanoemulsion, umfassend Polyoxypropylen-Polyoxyethylen-Vitamin E, dargestellt durch die folgende allgemeine Formel 1 als ein Emulgator und Polyacrylsäure- oder Polyacrylsäurederivat-Crosspolymer als ein Emulsionshilfsmittel:

[Allgemeine Formel 1]

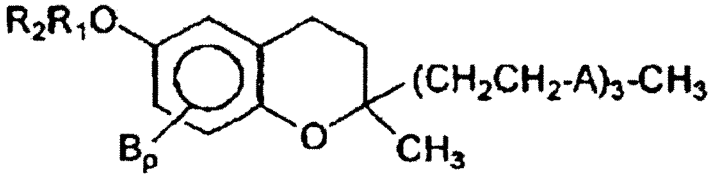

(1)

wobei $R_1$ $-(O-CH_2CH_2)_m-$ ist, m eine ganze Zahl von 0 bis 300 ist, $R_2$ $H(OCH(CH_3)CH_2)_n-$ ist, n eine ganze Zahl von 1 bis 250 ist; A

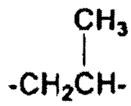

oder $-C(CH_3)=CH-$ ist; B an der 5-,7- oder 8-Position von Vitamin E $-CH_3$ ist; und p eine ganze Zahl von 1 bis 3 ist.

2. Nanoemulsion nach Anspruch 1, wobei die Polyacrylsäure- oder Polyacrylsäurederivat-Crosspolymere ein Carbomer, ein mit einem Allylether von Pentaerythritol, einem Allylether von Sucrose oder einem Allylether von Propylen vernetztes Homopolymer von Acrylsäure oder Acrylat-/$C_6$-$C_{40}$-Alkylacrylat-Crosscopolymere, Copolymer von $C_6$-$C_{40}$-Alkylacrylaten und einem oder mehreren Nanomeren von Acrylsäure, Methacrylsäure oder einer von deren einfachen Estern, vernetzt mit Allylether von Sucrose oder Allylether von Pentaerythritol, dargestellt durch die folgende allgemeine Formel 2 ist:

[Allgemeine Formel 2]

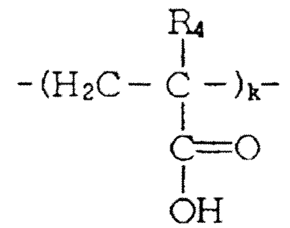

wobei $R_4$ H ader $CH_3$ ist, k eine ganze Zahl von 10 bis 100.000 ist.

3. Nanoemulsion nach Anspruch 2, wobei der Ether von Polyol ein Allylether von Pentaerythritol oder ein Allylether von Sucrose ist.

4. Nanoemulsion nach Anspruch 1, wobei das Vitamin E von Polyoxypropylen-Polyoxyethylen-Vitamin E ein natürliches Vitamin E oder ein synthetisches Vitamin E ist.

5. Nanoemulsion nach Anspruch 1, wobei der Gehalt an Polyoxypropylen-Polyoxyethylen-Vitamin E in der Gesamtnanoemulsion 0,5 bis 60 Gew.-% beträgt.

6. Nanoemulsion nach Anspruch 2, wobei der Gehalt des Polyacrylsäure- oder des Polyacrylsäurederivat-Crosspolymers der Gesamtnanoemulsion 0,01 bis 40 Gew.-% beträgt.

**7.** Nanoemulsion nach Anspruch 1, wobei der mittlere Tröpfchendurchmesser der Nanoemulsion im Bereich von 43 bis 96 nm liegt.

**8.** Kosmetische, nichttherapeutische Zusammensetzung, umfassend die Nanoemulsion nach Anspruch 1 bis Anspruch 7.

**9.** Kosmetische Zusammensetzung nach Anspruch 8, wobei die kosmetische Zusammensetzung ein Produkt ist, das gewählt ist aus der Gruppe von Creme- oder Lotionstypprodukten des Hautschutzes, der Hautatopieverbesserung, der Hautdepigmentierungs- und Hautfaltenverbesserung.

**10.** Zusammensetzung, umfassend eine Nanoemulsion nach einem der Ansprüche 1 bis 7 zur Verwendung bei einer therapeutischen Behandlung zur Behandlung der Haut, die gewählt ist aus Aknebehandlung.

**11.** Zusammensetzung zur Verwendung nach Anspruch 10, wobei die Zusammensetzung in einem Produkt ist, das gewählt ist aus der Gruppe, die besteht aus Creme- oder Lotionstypprodukten einer Aknebehandlung und Adrenalinkortexhormonprodukten.

**12.** Herstellungsverfahren einer kosmetischen Nanoemulsionszusammensetzung, wobei öllösliche Komponenten und wasserlösliche Komponenten vermischt werden mit Polyoxypropylen-Polyoxyethylen-Vitamin E, ausgedrückt in der allgemeinen Formel 1 als Emulgator und Polyacrylsäure- oder Polyacrylsäurederivat-Crosspolymer als Emulsionshilfsmittel:

[Allgemeine Formel 1]

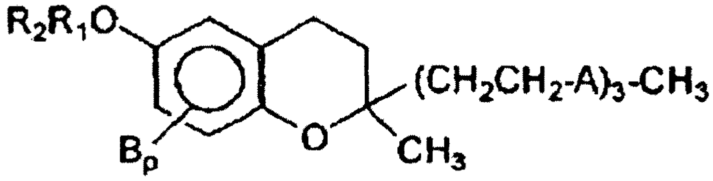

(1)

wobei $R_1$ $-(O\text{-}GH_2CH_2)_m-$ ist, m eine ganze Zahl von 0 bis 300 ist, $R_2$ $H(OCH(CH_3)CH_2)_n-$ ist, n eine ganze Zahl von 1 bis 250 ist; A

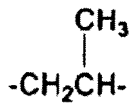

oder $-C(CH_3)=CH-$ ist; B an der 5-,7- oder 8-Position von Vitamin E $-CH_3$ ist; und p eine ganze Zahl von 1 bis 3 ist.

**13.** Herstellungsverfahren nach Anspruch 12, wobei die kosmetische Nanoemulsionszusammensetzung hergestellt wird durch Emulgieren des viskoelastischen Komplexes von öllöslicher Phase und wasserlöslicher Phase, wobei das Gewichtsverhältnis (ϕ) von öllöslicher Phase zu öllöslicher Phase plus wasserlöslicher Phase 0,4 bis 0,75 beträgt, und dann, wenn nötig, Wieder-Emulgieren des emulgierten viskoelastischen Komplexes nach Zugeben der restlichen öllöslichen Phase und wasserlöslichen Phase.

**14.** Herstellungsverfahren nach Anspruch 12 oder Anspruch 13, wobei die kosmetische Nanoemulsionszusammensetzung hergestellt wird durch Hochgeschwindigkeitsmischen des Komplexes von öllöslicher Phase und wasserlöslicher Phase bei einem θ-punkt über 40°C, wenn der vorige Komplex viskoelastische Eigenschaften zeigt.

**15.** Herstellungsverfahren nach Anspruch 14, wobei die kosmetische Nanoemulsionszusammensetzung hergestellt wird bei 1 atm durch einen Hochgeschwindigkeitsmischer, der gewählt ist aus der Gruppe, welche aus einem Propeller, einem Dispergierer, einem Schüttler und einem Homogenisierer besteht.

**Revendications**

1. Nanoémulsion comprenant de la polyoxypropyléne-polyoxyéthylène vitamine E représentée par la formule générale 1 suivante, comme émulsifiant, et un polymère réticulé d'acide polyacrylique ou de dérivé d'acide polyacrylique comme adjuvant d'émulsion :

[formule générale 1]

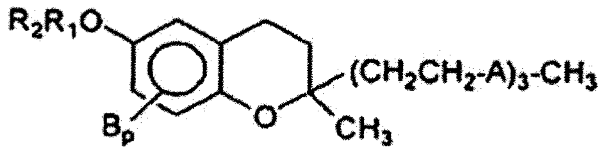

(I)

dans laquelle $R_1$ est $-(O-CH_2CH_2)_m-$, m est un entier de 0 à 300, $R_2$ est $H(OCH(CH_3)CH_2)_n-$, n est un entier de 1 à 250 ; A est

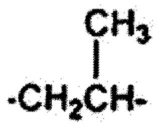

ou $-C(CH_3)=CH-$ ; B est $-CH_3$ à la position 5, 7 ou 8 de la vitamine E ; et p est un entier de 1 à 3.

2. Nanoémulsion selon la revendication 1, dans laquelle le polymère réticulé d'acide polyacrylique ou de dérivé d'acide polyacrylique est un carbomère, un homopolymère d'acide acrylique réticulé avec un allyléther de pentaérythritol, un allyléther de saccharose ou un allyléther de propylène ou un polymère réticulé acrylate/acrylate de $C_6$-$C_{40}$ alkyle, un copolymère d'acrylates de $C_6$-$C_{40}$ alkyle et d'un ou plusieurs nanomères d'acide acrylique, d'acide méthacrylique ou d'un de leurs esters simples réticulé avec un allyléther de saccharose ou un allyléther de pentaérythritol représenté par la formule générale 2 suivante :

[formule générale 2]

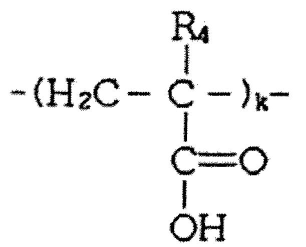

dans laquelle $R_4$ est H ou $CH_3$, k est un entier de 10 à 100 000.

3. Nanoémulsion selon la revendication 2, dans laquelle l'éther de polyol est un allyléther de pentaérythritol ou un allyléther de saccharose.

4. Nanoémulsion selon la revendication 1, dans laquelle la vitamine E de la polyoxypropylène-polyoxyéthylène vitamine E est une vitamine E naturelle ou une vitamine E synthétique.

5. Nanoémulsion selon la revendication 1, dans laquelle la teneur de palyoxypropylène-polyoxyéthylène vitamine E dans la nanoémulsion totale est 0,5 à 60 % en poids.

**6.** Nanoémulsion selon la revendication 2, dans laquelle la teneur du polymère réticulé d'acide polyacrylique ou de dérivé d'acide polyacrylique dans la nanoémulsion totale est 0,01 à 40 % en poids.

**7.** Nanoémulsion selon la revendication 1, dans laquelle le diamètre de gouttelette moyen de la nanoémulsion est dans la plage de 43 à 96 nm.

**8.** Composition cosmétique non thérapeutique comprenant la nanoémulsion selon la revendication 1 à la revendication 7.

**9.** Composition cosmétique selon la revendication 8, dans laquelle la composition cosmétique est un produit choisi dans le groupe des produits de type crème ou lotion de protection de la peau, d'amélioration de la peau atopique, de dépigmentation de la peau et d'amélioration des rides.

**10.** Composition comprenant une nanoémulsion selon l'une quelconque de la revendication 1 à la revendication 7 pour son utilisation dans un traitement thérapeutique pour traiter la peau choisi parmi le traitement de l'acné.

**11.** Composition pour son utilisation selon la revendication 10 dans laquelle la composition est un produit choisi dans le groupe des produits de type crème ou lotion d'un traitement de l'acné et d'hormone corticale adrénaline.

**12.** Procédé de préparation d'une composition cosmétique de type nanoémulsion dans lequel les composants solubles dans l'huile et les composants solubles dans l'eau sont mélangés avec de la polyoxypropylène-polyoxyéthylène vitamine E exprimée dans la formule générale 1 comme émulsifiant, et un polymère réticulé d'acide polyacrylique ou de dérivé d'acide polyacrylique comme adjuvant d'émulsion :

[formule générale 1]

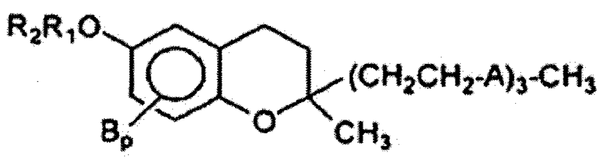

(I)

dans laquelle $R_1$ est $-(O-CH_2CH_2)_m-$, m est un entier de 0 à 300, $R_2$ est $H(OCH(CH_3)CH_2)_n-$, n est un entier de 1 à 250 ; A est

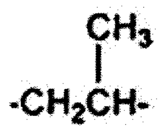

ou $-C(CH_3)=C_H-$ ; B est $-CH_3$ à la position 5, 7 ou 8 de la vitamine E ; et p est un entier de 1 à 3.

**13.** Procédé de préparation selon la revendication 12, dans lequel la composition cosmétique de type nanoémulsion est préparée par émulsification du complexe viscoélastique de phase soluble dans l'huile et de phase soluble dans l'eau, où le rapport en poids ($\phi$) de la phase soluble dans l'huile à la phase soluble dans l'eau plus la phase soluble dans l'eau est 0,4 à 0,75, puis, si nécessaire, de nouveau émulsification du complexe viscoélastique émulsifié après addition de la phase soluble dans l'huile et de la phase soluble dans l'eau restantes.

**14.** Procédé de préparation selon la revendication 12 ou la revendication 13, dans lequel la composition cosmétique de type nanoémulsion est préparée par mélange à grande vitesse du complexe de phase soluble dans l'huile et de phase soluble dans l'eau à un point $\theta$ au-dessus de 40°C quand le complexe précédent présente des propriétés viscoélastiques.

**15.** Procédé de préparation selon la revendication 14, dans lequel la composition cosmétique de type nanoémulsion

est préparée à une pression de 1 atmosphère par un mélangeur à grande vitesse choisi dans un groupe comprenant une hélice, un disperseur, un agitateur et un homogénéisateur.

【Figure 1】

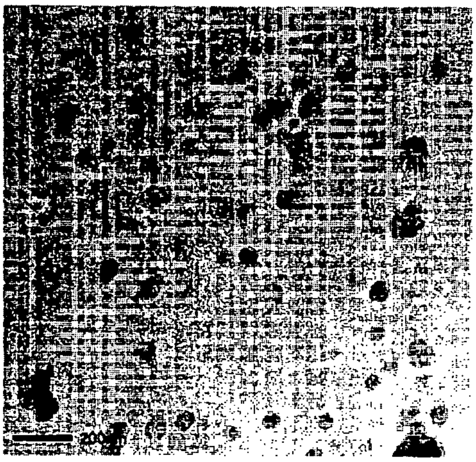


【Figure 2】

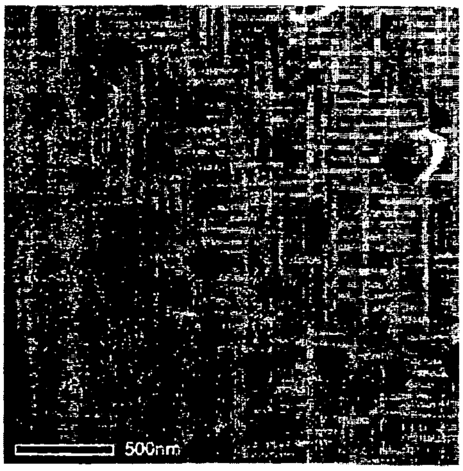

【Figure 3】

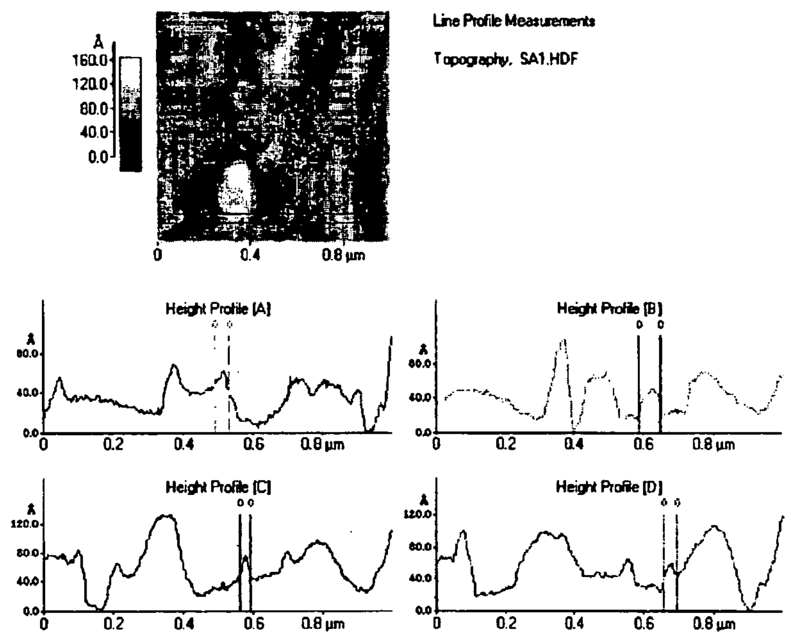


| Line | Height | Distance | Angle | Rp-v | Rms | Ave. | Mean Ht | Median Ht |
|---|---|---|---|---|---|---|---|---|
| [A] | 0: 15.8 Å | 0: 392 Å | 0: 2.3° | 98.3 Å | 15.7 Å | 12.4 Å | 70.0 Å | 70.1 Å |
| [B] | 0: 4.96 Å | 0: 627 Å | 0: 0.5° | [illegible] | 19.9 Å | 15.6 Å | 70.1 Å | 70.1 Å |
| [C] | 0: 12.4 Å | 0: 314 Å | 0: 2.3° | 133 Å | 30.2 Å | 24.3 Å | 70.1 Å | 67.6 Å |
| [D] | 0: 2.31 Å | 0: 352 Å | 0: 0.3° | 121 Å | 27.7 Å | 23.6 Å | 70.1 Å | 67.9 Å |
| Ave. | 8.88 Å | 431 Å | 1.3° | 116 Å | 23.4 Å | 19.0 Å | 70.1 Å | 68.9 Å |

【Figure 4】

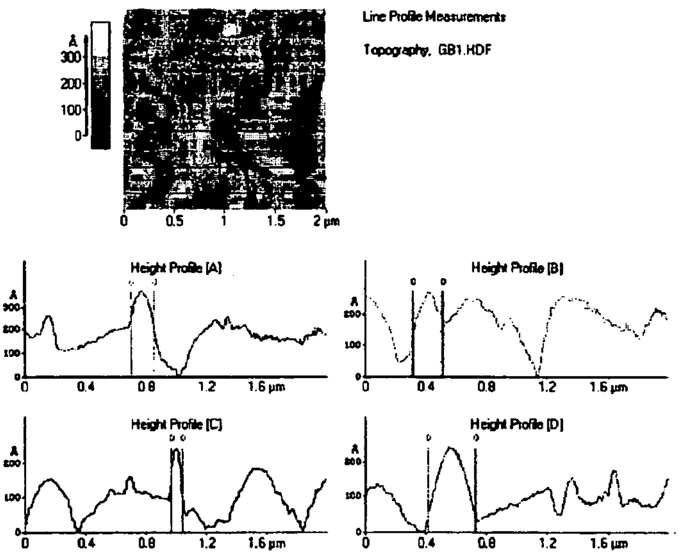


| Line | Height | Distance | Angle | Rp-v | Rms | Ave | Mean Ht | Median Ht |
|---|---|---|---|---|---|---|---|---|
| [A] | [illegible] | [illegible] | [illegible] | 364 Å | 67.4 Å | 47.8 Å | 190 Å | 189 Å |
| [B] | 0: 31.2 Å | 0: 0.204 um | 0: 0.9° | [illegible] | [illegible] | [illegible] | [illegible] | [illegible] |
| [C] | 0: 110 Å | 0: 784 Å | 0: 0.0° | 243 Å | 52.0 Å | 42.2 Å | 190 Å | 192 Å |
| [D] | 0: 23.5 Å | 0: 0.314 um | 0: 0.4° | 239 Å | 49.7 Å | 37.2 Å | 190 Å | 182 Å |
| Ave. | 65.4 Å | 0.186 µm | 3.3° | 280 Å | 57.8 Å | 44.1 Å | 190 Å | 191 Å |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 488220
- WO 9318752 A
- KR 20000000840


### Non-patent literature cited in the description


- **Flockhart, I. R.** Nanoemulsions derived from lanolin show promising drug delivery properties. *J. Pharm. Pharmacol.,* 1998, vol. 50, 141
- **Young-Dae Kim.** *Novel Nanoemulsions of POP-POE Tocopheryl Ether,* 14 October 2002
- **Young-Dae Kim.** *A Novel Nanoemulsification Method: Stirring of a Mixture of Tocopherol-Containing Block Co-Polymer Emulsifier at θ-Point,* 24 October 2004
- **Young-Dae Kim.** *The Formation and Stability of Liquid Nanoemulsions from Tocopherol Derivative Nonionic Amphiphiles,* 24 September 2002
- **Young-Dae Kim.** *A Novel Nanoemulsification Method of Stirring at the θ-Point with the Tocopherol-Based Block Copolymer Nonionic Emulsifier PPG-20 Tocophereth-50,* September 2004, vol. 7 (4), 319-326